Europäisches Patentamt

European Patent Office

Office européen des brevets

(19)

(11) Publication number: **0 039 528**

**B1**

(12) **EUROPEAN PATENT SPECIFICATION**

(45) Date of publication of patent specification: **15.06.83**

(21) Application number: **81200447.1**

(22) Date of filing: **23.04.81**

(51) Int. Cl.³: **A 23 J 3/00,** A 23 L 1/20, A 61 K 37/02

(54) **Process for preparing soya proteins of agreeable taste for human use and product thus obtained.**

(30) Priority: **02.05.80 IT 2175380**

(43) Date of publication of application:
**11.11.81 Bulletin 81/45**

(45) Publication of the grant of the patent:
**15.06.83 Bulletin 83/24**

(84) Designated Contracting States:
**AT BE CH DE FR GB LI LU NL SE**

(56) References cited:
**FR - A - 2 287 856**
**US - A - 3 865 956**

(73) Proprietor: **Ferrero, Aldo**
**Via Ampere, 9**
**I-20131 Milano (IT)**

(72) Inventor: **Ferrero, Aldo**
**Via Ampere, 9**
**I-20131 Milano (IT)**

(74) Representative: **Giambrocono, Alfonso, Dr. Ing.**
**et al,**
**Ing. A. Giambrocono & C. s.r.l. Via Rosolino Pilo,**
**19/b**
**I-20129 Milano (IT)**

Courier Press, Leamington Spa, England.

Process for preparing soya proteins of agreeable taste for human use and product thus obtained

This invention relates to a process for preparing palatable soya proteins for direct human consumption and a product thus obtained. The high alimentary and dietetic value of the derivatives of soya seeds which are rich in vegetable proteins is known for a long time. In the prior art many processes are described for obtaining such products. The processes have the purpose of removing most of the fatty materials and not the protein soluble components. The product obtained is usually referred as T.S.P. (Textured Soya Protein) or T.V.P. (Textured Vegetable Proteins), Textured in this connection means manufactured by thermoplastic extrusion and not by a spinning process. Such T.V.P. are not considered suitable for consumption as "ready as such" product for human consumption, i.e. that it can not be chewed and ingested as such because of being not aggreeable as regards taste. Therefore, such soya proteins are used in various percentages as additives mixed with various food such as meat, flour and the like, to prepare cooked food, such as hamburgers, wurstels, and baked products, such as sweets, bread and the like.

This invention relates to a process allowing the manufacture of products suitable for the consumption of the T.V.P. soya proteins, ready as such, because of giving an agreeable taste thereto, so that such soya proteins can be chewed and ingested without any further preparation thereof; or that they can be readily added to food, such as broth, milk, tea, yoghurt, and still many other not solid food, by a simple instantaneous dispersing operation in the preselected food. This is very important, particularly for curative treatments.

The original product under examination has a protein content on dry solids (N×6,25) of about 50%; however, soya proteins with a higher protein content up to about 90% on dry solids can also be used. As to particle size, the soya proteins recognized as most suitable for use according to the present process are those in the form of irregular granules of about the following size: max. 5% larger than 5,0 mm, min. 85% between 1.2 and 5.0 mm, maw 7% less than 0,5 mm; of course larger or smaller sizes can also be used, but it was found that within the above specified size range the process is most efficient and the consumption use most suitable.

The subject process of the present invention has the purpose of subjecting the soya proteins, preferably in the form of extruded granules, to a dry baking process, preferably in hot air driers, at a temperature in the range of 40°C to 120°C for a time between 3 and 14 hours. Surprisingly, after such a dry baking process, the granules take an agreeable taste, having changed the organoleptic characteristics thereof which could not have been foreseen beforehand since the original product had been previously heat processed at high temperature (of about 120°C or higher) as a result of the extrusion operation.

The process according to the invention will become apparent from the following description: The raw material f.e. are commercially available T.V.P. granules, also known under the trademark "Textratein", manufactured by CARGILL (U.S.A.). A commercially available type of drier such as the commonly used for the drying of alimentary pastes of small size (soup pastes) was used for dry baking; the drier consists of a closed cabinet, having therein movable frames with narrow mesh grates. The meshes of the frame grates should be narrow enough to hold the granules of the above mentioned size, preventing the granules from outflowing from the meshes, and the frame are movable for easy loading and unloading thereof after T.V.P. drying.

Conveniently, the drier can be an electrically operated drier. Of course, a similar result can be provided by using continuous driers, or suitably adapted ovens, or by further similar expedients. The optimum drying temperature is between 60°C and 90°C, whereas the optimum drying time is in the range of 5 to 10 hours.

The grid frames are filled with T.V.P. granules of soya up to a thickness of 1 to 5 centimeters, so that the hot air stream can freely circulate therethrough. Prior to processing the proteins to be baked have a moisture content of about 10%. After baking, the moisture content thereof is about 5% on the average, but it may occur that such a moisture content is lower and even slightly higher than such an average value. After the optimum baking period of about 5—10 hours which is sufficient for achieving the organoleptic change the grid frames are removed and emptied. The product can be sieved to remove the pulverulent material but this operation is not necessarily required. The T.V.P. granules have already acquired the characteristics of desirable taste as above described.

Thus, while being rejected as such prior to processing due to the not agreeable taste thereof, following said baking step the T.V.P. soya granules become of acceptable taste, or agreeable to the palate, and accordingly are suitable for consumption as a ready to use product without any further modifications or preparations.

The improvement in the organoleptic taste qualities of the T.V.P. granules is not due to loss of moisture, but to the variation occurring as a result of the dry baking step to which such granules have been subjected at a not unduly temperature and this after the treatment at high temperature previously applied thereto as a result of the extrusion operation.

Moreover, for substantial curative purposes, even if still other results are at the same time achieved, such as a partial impermeabilization thereof, the improvement in taste thereof and that of rendering the T.V.P. soya granules of a more conventional agreeable appearance by taking a colour similar to that of hazel-nut granules, an appearance characteristic not to be neglected in a food to be ingested as such, the T.V.P. granules are preferably impregnated with soya lecithin in a percentage ranging between 5% and 20% by weight based on the amount of the dry granules.

Thus their processing is very important, since said T.V.P. granules are particularly intended for curative use, such as the treatment of hyper-cholestereamias. The enrichment thereof in soya lecithin increases their characteristics of suitability to this purpose.

It is known that not only vegetable proteins, and particularly soya proteins are highly suitable for therapeutic treatments also as lypo-stabilizer but also other soya derivatives such as phospholipids are of curative value. It is also known that soya lecithin particularly performs the function of dispersing the blood flowing fatty materials, making such materials more soluble. The soya lecithin used in the present process is of alimentary standard type containing about 33% of soya oil in semi-thick form. The pure soya lecithin without soya oil is not used even if its adoption in the present process is practically possible because such type of soya lecithin is subjected to autoxydation and to the action of microorganisms and enzymes. Its stability is doubtful, so that during long periods of storage it could compromise the shelf-life of the soya proteins enriched with soya lecithin. The type used appears to be a slippery and greasy and repellant to palate food which cannot be in any case considered suitable as such for the direct human consumption, even in very small doses.

By processing granulated T.V.P. proteins as dry baked according to the invention with soya lecithin in paste form even at high percentages at a temperature such that the characteristics thereof are not impaired or destroyed (lecithins) are destroyed by heat at 100°C for a time of 30 minutes), the goal is achieved to emphasize the curative characteristics thereof in addition to the above mentioned other advantages.

In order to achieve such a result after being dry baked said soya protein granules are placed within a fixed rotating blade drum or cylinder or any other vessel suitable for this purpose. As the blades are rotating, the paste soya lecithin is very slowly poured on such granules; the soya lecithin particularly in winter or at low temperatures will be suitably pre-heated in order to make it more flowable since, if too thick, the dispersion thereof is difficult if not impossible. T.V.P. granules will readily be impregnated by the soya lecithin, the latter perfectly incorporating therein. Surprisingly, the granules so enriched with soya lecithin improve more in their taste, becoming as such more agreeable if chewed and ingested than the one not enriched with soya lecithin. Moreover, the agreeable taste is even strengthened during storage. One has available, in this manner, a finished product which does not conflict with the long periods of normal storage, and showing an almost unlimited shelf-life without organoleptic variation.

Such process of the granules can also be made before their dry baking but a good production performance suggests to do if after the dry baking. Even at the high amount or dose of 10% of lecithin, which is considered as optimum for curative purposes, traces of greasiness or residues are not left on the granules surface. Of course, the percentage of soya lecithin can be varied also at substantially higher or lower values than the above stated optimum value of 10%. It is essential that the soya lecithin can easily be taken up by the protein granules and that the taste resulting therefrom is suitable.

It is particularly outlined that a dual result is thus obtained, namely of giving a higher dietetic-curative power to the soya protein granules enriched with a high percentage of an efficient phospholipid, that is soya lecithin which yields an agreeable and ready-to-use product.

The soya T.V.P. granules according to the invention are ready for sale for direct human consumption as such in a very wide alimentary-dietetic field, particularly for curative purposes.

According to the invention it is possible to have at disposal T.V.P. soya proteins in the form of granules, further enriched with soya lecithin easy to use which may be available in suitably dosed amounts for daily ingestion. Such proteins can be ingested as such both by chewing or instantaneous dispersion thereof in any not solid food. The daily dose of the product of the invention contains about 30—80 g, preferably 50—60 g.

Finally, for persons of particular palate, the taste of such T.V.P. granules with or without lecithin can be further modified by adding still at a very slow rate to said rotating blade drum or cylinder either fluid hazelnut paste, in case even sweetened, or other fluid or made fluid ingredient suitable for human nourishment or food.

Of course, on the basis of the above described elements, the inventive process can be varied as desired, even omitting the use of soya lecithin, or replacing it for consumption in confectionery-dietetic field with other suitable ingredients of fluid or semi-fluid both sweetened or in case even salted state.

Example 1

The raw material used in the process was a textured soya protein in granular form (at least 85% of the granules between 1,2 and 5 mm) commercially available under the trademark "Textratein" (manufactured by CARGILL) with the following main ingredients: about 50% raw protein, 1.5% fat (ether extract), 10% moisture content, 6.5% minerals (ash), 4.5% raw fiber.

The equipment used was an electric drier of the type Barcaccia Mod ETB/40, provided with 40 movable wooden frames sized 100×50 cm with stainless steel nets.

4 kg of the raw material was placed into each of the frames and the frames are put into the electric drier. At a drying temperature of 80°C, the granules are dried for 7 hours without interruption. After this time the frames are removed from the drier and emptied. The product was sieved with a sieve with suitable meshes to eliminate the powder formed during the drying operation. During the drying the moisture content of the product was reduced to about 2—3%. From 160 kg of the raw material, 149 kg of the finished product was obtained.

Example 2
The method of Example 1 was employed. After the drying operation 100 kg of the product was put into a stainless steel container with rotating blades, having a filling-hole on the top and a rolling shutter below to unload the product after the operation.

Soya lecithin of the alimentary standard type containing about 33% of soya oil, 21% of choline lecithin, 20% of inositole phosphatides, 11% of phosphadylserine and other phosphatides, 8% of phosphatides lecithin, 5% of carbohydrates and 2% of sterin and tocopherol was heated in a drier for about 3 hours at 60°C in order to make it more fluid.

10 kg of the warmed soya lecithin were poured very slowly (during about 5 minutes) into the container with rotating blades. Subsequently, always by rotating blades, the process was continued for further 15 minutes. After some days of storage the granules impregnated with soya lecithin lost the greasy appearence; the lecithin was perfectly absorbed by the soya protein granules. The product was ready for packing.

Claims

1. A process for preparing soya proteins of agreeable taste for human use, from defatted or scoured soya flour and/or soya proteins, characterized in that soya flour and/or soya proteins in the form of granules are subjected to a dry baking step at a temperature in the range of 40°C to 120°C for a time between 3 and 14 hours.

2. A process according to claim 1 wherein the starting material is an extruded soya protein in the form of granules.

3. A process according to the preceding claims, characterized in that the baking step is carried out in hot air driers at an optimum temperature ranging between 60°C and 90°C for 5—10 minutes.

4. A process according to the preceding claims, characterized in that the soya protein before or after the baking step is impregnated with 5% to 20% by weight of soya lecithin based on the amount of the dry granules.

5. Product obtained from defatted or scoured, extruded soya flour and/or soya proteins in granular or in powder form, obtained by the process according to the preceding claims.

6. Product according to claim 5 enriched with 5% to 20% by weight of soya lecithin based on the amount of the dry granules.

Revendications

1. Procédé pour la préparation de protéines de soja de goût agréable pour la consommation humaine, à partir de farine de soja déshuilée ou dessuintée et/ou de protéines de soja, caractérisé en ce que de la farine de soja et/ou des protéines de soja sous forme de granulés sont soumis à une phase de cuisson à sec, à une température de l'ordre de 40 à 120 degrés centigrades durant un laps de temps se situant entre 3 et 14 heures.

2. Procédé selon la revendication 1, caractérisé en ce que le matériau initial est une protéine de soja extrudée sous forme de granulés.

3. Procédé selon les revendications précédentes, caractérisé en ce que la phase de cuisson est exécutée dans des séchoirs à air chaud, à une température optimale se situant entre 60 et 90 degrés centigrades, pendant 5 à 10 heures.

4. Procédé selon les revendications précédentes, caractérisé en ce que la protéine de soja, avant ou après la phase de cuisson, est imprégnée avec 5 à 20 pour cent en poids de lécithine de soja, la proportion étant basée sur la quantité des granulés secs.

5. Produit obtenu à partir de farine de soja extrudée, déshuilée ou dessuintée et/ou de protéine de soja sous forme granuleuse ou pulvérulente, le produit étant obtenu au moyen du procédé selon les revendications précédentes.

6. Produit selon la revendication 5, caractérisé en ce qu'il est enrichi de 5 à 20 pour cent en poids de lécithine de soja, la proportion étant basée sur la quantité de granulés secs.

Patentansprüche

1. Verfahren zum Bereiten von Soyaprotein angenehmen Geschmackes für den menschlichen Gebrauch, wobei von entfettetem oder gereinigtem Soyamehl und/oder Soyaprotein ausgegangen wird, dadurch gekennzeichnet, daß das Soyamehl und/oder Soyaprotein in körniger Form in einem Temperaturbereich von 40°C bis 120°C während 3 bis 14 Stunden getrocknet bzw. gedörrt wird.

2. Verfahren nach Anspruch 1, dadurch gekennzeichnet, daß das Ausgangsmaterial extrudiertes Soyaprotein in körniger Form ist.

3. Verfahren nach den vorstehenden Ansprüchen, dadurch gekennzeichnet, daß das Trocknen bzw. Dörren in Heißlufttrocknern bei einer Temperatur zwischen 60°C und 90°C während 5 bis 10 Stunden durchgeführt wird.

4. Verfahren nach den vorstehenden Ansprüchen, dadurch gekennzeichnet, daß das Soyaprotein vor oder nach dem Trocknen bzw. Dörren mit 5 bis 20 Gewichtsprozent Soyalecithin, bezogen auf die Menge an trockenen Körnern, getränkt wird.

5. Produkt aus entfettetem oder gereinigtem, extrudiertem Soyamehl und/oder Soyaprotein in Form von Körnern oder Mehl hergestellt nach dem Verfahren nach den vorstehenden Ansprüchen.

6. Produkt nach Anspruch 5, dadurch gekennzeichnet, daß es angereichert ist mit 5 bis 20 Gewichtsprozent Soyalecithin, bezogen auf die Menge an trockenen Körnern.